# EUROPEAN PATENT APPLICATION

(11) **EP 4 599 763 A1**
(43) Date of publication of application: **13.08.2025**
(21) Application number: 23874835.4
(22) Date of filing: 02.10.2023
(51) Int. Cl.: A61B 5/11, G01C 19/5776, G01P 15/18

(54) **INFORMATION PROCESSING DEVICE AND INFORMATION PROCESSING METHOD**

(30) Priority: 04.10.2022 JP 2022160564
(71) Applicant: Kyocera Corporation, Kyoto-shi, Kyoto 612-8501 (JP)
(72) Inventor: ARIMURA TOKAIRIN, Shiori, Kyoto-shi, Kyoto 612-8501 (JP); KIMPARA, Hideyuki, Kyoto-shi, Kyoto 612-8501 (JP); NAGATOMO, Takashi, Kyoto-shi, Kyoto 612-8501 (JP); KLINKIGT, Martin, Kyoto-shi, Kyoto 612-8501 (JP); HOSHUYAMA, Osamu, Kyoto-shi, Kyoto 612-8501 (JP); KISHI, Masayuki, Kyoto-shi, Kyoto 612-8501 (JP); YAMAMOTO MURAKAMI, Edwardo Arata, Kyoto-shi, Kyoto 612-8501 (JP); NISHIDA, Naoki, Kyoto-shi, Kyoto 612-8501 (JP); GOTO, Tomoya, Kyoto-shi, Kyoto 612-8501 (JP)
(74) Representative: Viering, Jentschura & Partner mbB Patent- und Rechtsanwälte
(86) International application number: PCT/JP2023/035970
(87) International publication number: WO 2024/075708

(57) **Abstract**

An information processing device includes a communication unit and a control unit. An acquisition unit acquires an original acceleration and an original angular velocity from a 3-axis inertial sensor. The control unit certifies start of one step using a first angle and a resultant value. The first angle is an angle with respect to a vertical direction based on the original angular velocity. The resultant value is based on the original acceleration.

## Description

### CROSS-REFERENCE TO RELATED APPLICATIONS

The present application claims priority from Japanese Patent Application No. 2022-160564 filed on October 4, 2022, the entire contents of which are hereby incorporated by reference.

### TECHNICAL FIELD

The present disclosure relates to an information processing device and an information processing method.

### BACKGROUND OF INVENTION

Provision of useful information through analysis of a gait state of a user is required. For the analysis of the gait state, accurate detection of the operation of the user during gait is needed. For example, a technique, such as motion capture using an inertial measurement unit and multiple cameras, is proposed to accurately detect the operation of the user (refer to Patent Literature 1).

### CITATION LIST

### PATENT LITERATURE

Patent Literature 1: Japanese Unexamined Patent Application Publication No. 2020-201183

### SUMMARY

In a first aspect, an information processing device includes an acquisition unit and a control unit.

The acquisition unit acquires an original acceleration and an original angular velocity in a local coordinate system based on a posture of a 3-axis inertial sensor from the inertial sensor.

The control unit certifies start of one step in gait of a wearer of the inertial sensor using a first angle with respect to a vertical direction based on the original angular velocity and a resultant value based on the original acceleration.

In a second aspect, an information processing method includes
acquiring an original acceleration and an original angular velocity in a sensor coordinate system from a 3-axis inertial sensor; and
detecting start of one step in gait of a wearer of the inertial sensor using a first angle with respect to a vertical direction based on the original angular velocity and a resultant value based on the original acceleration.

### BRIEF DESCRIPTION OF THE DRAWINGS

FIG. 1 is a schematic external view illustrating a use mode of an information processing system including an information processing device according to one embodiment.
FIG. 2 is a block diagram illustrating a schematic configuration of the information processing system in FIG. 1.
FIG. 3 is a diagram for describing a local coordinate system and a global coordinate system in the information processing system in FIG. 2.
FIG. 4 is a partial enlarged view of a graph indicating the relationship of a first angle and a resultant value with respect to change in time for describing a method of detecting a first time, a second time, and a third time.
FIG. 5 is a graph indicating the relationship of the first angle and the resultant value with respect to change in time for describing a first condition.
FIG. 6 is a graph indicating the relationship of the resultant value with respect to change in time for describing a second condition.
FIG. 7 is a flowchart for describing a certification process performed by a control unit in the information processing device in FIG. 2.
FIG. 8 is a flowchart for describing an exclusion process performed by the control unit in the information processing device in FIG. 2.

### DESCRIPTION OF EMBODIMENTS

Embodiments of the present disclosure will be described with reference to the drawings. The same signs are added to the same components, among the components illustrated in the following drawings.

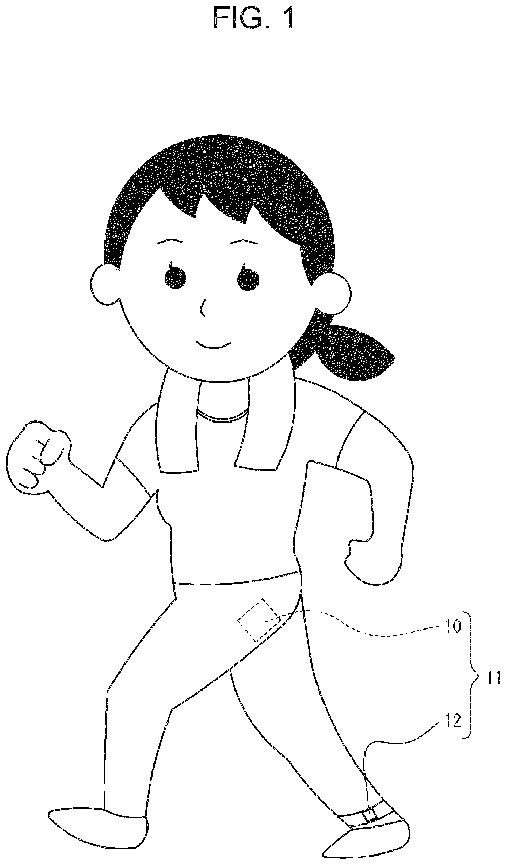

As illustrated in FIG. 1, an information processing system 11 including an information processing device 10 according to an embodiment of the present disclosure includes a sensor apparatus 12 and the information processing device 10. The sensor apparatus 12 may be of a type mounted to a leg of a wearer and, more specifically, may be a type mounted to an ankle of the wearer. The sensor apparatus 12 may detect an angular velocity and an acceleration of each of three axes directions as an original angular velocity and an original acceleration, respectively. The sensor apparatus 12 may transmit the original angular velocity and the original acceleration that are detected to the information processing device 10 as information. The information processing device 10 may analyze an operation of the wearer based on the original angular velocity and the original acceleration that are acquired.

The sensor apparatus 12 is, for example, an IMU (inertial measurement unit). The sensor apparatus 12 may be mounted near an ankle of the wearer in any mode. The sensor apparatus 12 may be built in, for example, a shoe, a sock, an insole, an anklet, or the like. The sensor apparatus 12 may be capable of being mounted to the ankle with a band, a clip, or the like.

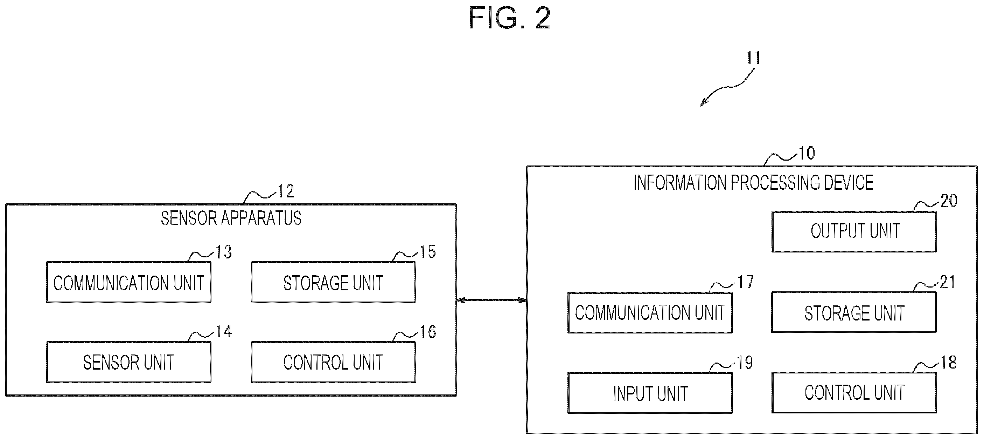

As illustrated in FIG. 2, the sensor apparatus 12 may include a communication unit 13, a sensor unit 14, a storage unit 15, and a control unit 16.

The communication unit 13 may include at least one communication module capable of communicating with the information processing device 10 via, for example, a wired or wireless communication line. The communication module is a communication module conforming to the standard of the communication line. The standard of the communication line is, for example, a short-range wireless communication standard, such as the Bluetooth (registered trademark) standard, an infrared radiation standard, or the NFC (near field communication) standard.

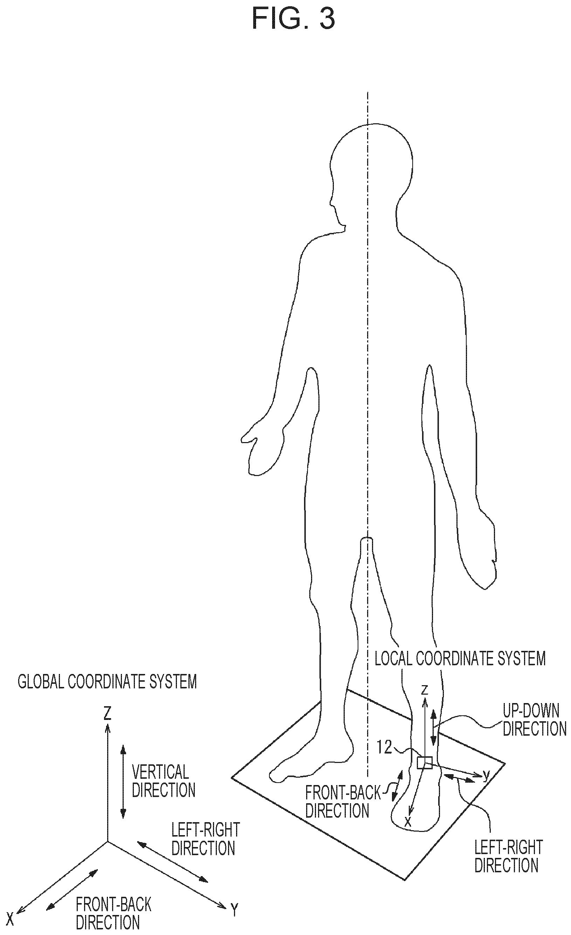

The sensor unit 14 includes an inertial sensor having at least three axes. The 3-axis inertial sensor includes, for example, a 3-axis acceleration sensor and a 3-axis gyro sensor. The 3-axis inertial sensor detects accelerations of the three axes and angular velocities of the three axes in a local coordinate system as the original accelerations and the original angular velocities, respectively. As illustrated in FIG. 3, the local coordinate system is a coordinate system based on a wearing posture of the sensor apparatus 12 when the sensor apparatus 12 is worn by the wearer. The local coordinate system is composed of, for example, the x axis, the y axis, and the z axis. The x axis, the y axis, and the z axis are orthogonal to each other. An ideal wearing posture is supposed for the sensor apparatus 12. In a state in which the sensor apparatus 12 is worn by the wearer in the wearing posture, the x axis is perpendicular to the direction in which a tibia extends and to the rotation axis of a talocrural joint and, in general, the x axis is parallel to the front-back direction with respect to the tibia. The y axis is perpendicular to the direction in which the tibia extends and to the x axis and, in general, the y axis is parallel to the left-right direction with respect to the tibia. The z axis is perpendicular to the x axis and the y axis and, in general, the z axis is toward the direction in which the tibia extends.

Referring to FIG. 2, the storage unit 15 may include any of a semiconductor memory, a magnetic memory, and an optical memory. The semiconductor memory is, for example, a RAM (random access memory), a ROM (read only memory), or the like. The RAM is, for example, an SRAM (static random access memory), a DRAM (dynamic random access memory), or the like. The ROM is, for example, an EEPROM (electrically erasable programmable read only memory) or the like. The storage unit 15 may function as a main storage, an auxiliary storage, or a cache memory. The storage unit 15 may store data used for the operation of the sensor apparatus 12 and data acquired by the operation of the sensor apparatus 12. The storage unit 15 stores, for example, a system program, an application program, built-in software, and so on.

The control unit 16 may include at least one processor, at least one dedicated circuit, or a combination of the at least one processor and the at least one dedicated circuit. The processor may be a general-purpose processor, such as a CPU (central processing unit) or a GPU (graphics processing unit), or a dedicated processor specialized for a specific process. The dedicated circuit may be, for example, an FPGA (field-programmable gate array), an ASIC (application specific integrated circuit), or the like. The control unit 16 may perform processes concerning the operation of the sensor apparatus 12 while controlling the respective components in the sensor apparatus 12.

The control unit 16 may acquire, for example, an instruction to start detection from the information processing device 10 via the communication unit 13. Upon acquisition of the instruction, the control unit 16 may cause the sensor unit 14 to start the detection of the original accelerations and the original angular velocities. The control unit 16 may acquire the original accelerations and the original angular velocities from the sensor unit 14. The control unit 16 may recognize the vertical direction in a global coordinate system based on the original angular velocities that are acquired since the instruction is acquired until an initialization time elapses. The wearer of the sensor apparatus 12 may be required to keep an initial posture, for example, an upright state during a time period since the instruction to start the detection is transmitted until the initialization time elapses.

As illustrated in FIG. 3, the global coordinate system is a coordinate system based on a position in a space in which the wearer wearing the sensor apparatus 12 walks. The global coordinate system is composed of, for example, the x axis, the y axis, and the z axis. The x axis, the y axis, and the z axis are orthogonal to each other. The x axis is parallel to the front-back direction of the wearer. The y axis is parallel to the left-right direction of the wearer. The z axis is parallel to the vertical direction. The coordinate axes in the global coordinate system in a correct posture of the wearer who wears the sensor apparatus 12 in the ideal wearing posture may be parallel to the corresponding coordinate axes in the local coordinate system. In the description of the present application, the correct posture of the wearer is a posture in which the direction in which a foot region extends in the upright state is kept so as to be parallel to the front-back direction.

The control unit 16 may control the communication unit 13 so as to transmit the original accelerations and the original angular velocities that are acquired to the information processing device 10 as signals after the recognition of the vertical direction. The control unit 16 may perform the acquisition and the transmission of the original accelerations and the original angular velocities at a predetermined time interval since then. The time interval may be set based on the waking speed or the like of a general user.

As illustrated in FIG. 2, the information processing device 10 includes a communication unit (an acquisition unit) 17 and a control unit 18. The information processing device 10 may further include an input unit 19, an output unit 20, and a storage unit 21.

The communication unit 17 may include at least one communication module capable of communicating with the sensor apparatus 12 via, for example, a wired or wireless communication line. The communication module is a communication module conforming to the standard of the communication line. The standard of the communication line is, for example, a short-range wireless communication standard, such as the Bluetooth (registered trademark) standard, an infrared radiation standard, or the NFC standard. The communication unit 17 acquires the original accelerations and the original angular velocities from the sensor apparatus 12.

The input unit 19 may include at least one input interface that detects an operation input by the user. The input interface is, for example, physical keys, electrostatic capacitance keys, a pointing device, a touch screen integrally provided with a display of the output unit 20, a microphone, or the like.

The output unit 20 may include at least one output interface that outputs information to notify the user of the information. The output interface is, for example, a display that outputs information as a video, a speaker that outputs information as a sound, or the like. The display is, for example, an LCD (liquid crystal display), an organic EL (electro luminescence) display, or the like.

The storage unit 21 may include any of a semiconductor memory, a magnetic memory, and an optical memory. The semiconductor memory is, for example, a RAM, a ROM, or the like. The RAM is, for example, an SRAM, a DRAM, or the like. The ROM is, for example, an EEPROM or the like. The storage unit 21 may function as a main storage, an auxiliary storage, or a cache memory. The storage unit 21 may store data used for the operation of the information processing device 10 and data acquired by the operation of the information processing device 10. The storage unit 21 stores, for example, a system program, an application program, built-in software, and so on.

The control unit 18 may include at least one processor, at least one dedicated circuit, or a combination of the at least one processor and the at least one dedicated circuit. The processor may be a general-purpose processor, such as a CPU or a GPU, or a dedicated processor specialized for a specific process. The dedicated circuit may be, for example, an FPGA, an ASIC, or the like. The control unit 18 may perform processes concerning the operation of the information processing device 10 while controlling the respective components in the information processing device 10.

The control unit 18 certifies a start time of one step (start of one step) in gait of the wearer of the sensor apparatus 12 using a first angle and a resultant value based on the original angular velocities and the original accelerations, respectively, which are acquired in the communication unit 17. In addition, the start time certified by the control unit 18 may be a start time of one step in normal gait. Detection of the start time, which is performed by the control unit 18, will be described in detail below.

The first angle may be the angle of the z axis of the sensor apparatus 12 with respect to the vertical direction. Alternatively, the first angle may be the angle of the direction in which the tibia extends with respect to the vertical direction in the global coordinate system. The resultant value is the magnitudes of the original accelerations, that is, the magnitude of a resultant vector of the original acceleration components of the three axes.

In the description of the present application, the start time of one step in gait is a time (point of time) at which the foot that is moved forward lands on the ground or the like. The normal gait is a continuous gait state, that is, a moving operation excluding states in which both feet are apart from the ground, such as running, jumping, and skipping, and an operation excluding unsteady gait, such as stepping.

The control unit 18 may convert the original accelerations and the original angular velocities that are acquired into the accelerations and the angular velocities in the global coordinate system based on change in posture with respect to the initial posture. The control unit 18 may calculate a rotation matrix for converting the local coordinate system into the global coordinate system from the difference between the original accelerations in measurement and the original accelerations at the initial posture and the difference between the original angular velocities in measurement and the original angular velocities at the initial posture, for example, based on the initial posture. The control unit 18 may convert the original accelerations and the original angular velocities into the accelerations and the angular velocities, respectively, in the global coordinate system using the rotation matrix.

The control unit 18 may perform integration of the acquired original angular velocities to calculate the posture of the sensor apparatus 12 in the local coordinate system. The posture in the local coordinate system may be represented by the rotation angle with respect to the three axes in the local coordinate system. The posture in the local coordinate system may include the angle of the z axis with respect to the vertical direction. Alternatively, the control unit 18 may perform integration of the angular velocities resulting from the conversion to calculate the posture of a wearing portion of the wearer who wears the sensor apparatus 12 in the global coordinate system. The posture in the global coordinate system may be represented by the rotation angle with respect to the three axes in the global coordinate system. The posture in the global coordinate system may include the angle of the direction in which the tibia extends with respect to the vertical direction. Accordingly, the control unit 18 may calculate the first angle.

The control unit 18 may compute geometric mean of the original acceleration components of the three axes to calculate the resultant value. Alternatively, the control unit 18 may compute geometric mean of the acceleration components of the three axes, which results from the conversion, to calculate the resultant value.

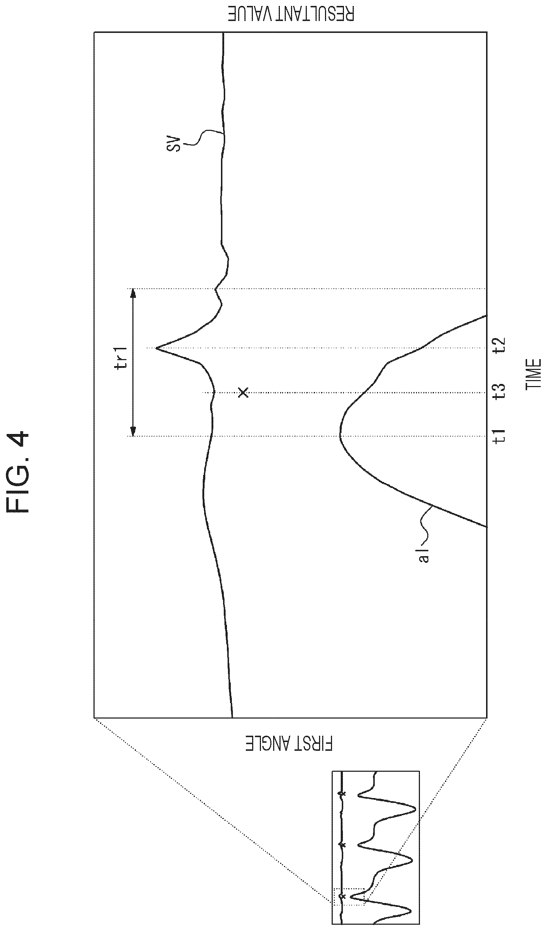

As illustrated in FIG. 4, the control unit 18 may detect a first time (a first point of time) t1 at which a first angle al that is varied with time is locally maximized. The control unit 18 may detect a second time (a second point of time) t2 at which a resultant value sv is locally maximized within a first time range tr1 from the first time t1. The first time range tr1 is a supposed time since the foot is swung forward and up until the foot lands on the ground and is, for example, within a range that is longer than or equal to zero seconds and that is shorter than or equal to 100 m. The control unit 18 may detect a third time (a third point of time) t3 at which the resultant value sv is locally minimized toward the first time t1 from the second time t2. The control unit 18 may certify the third time t3 as the start time of one step in gait.

The control unit 18 may exclude the detected third time t3 from the certification of the start time of one step in normal gait if at least one of a first condition or a second condition, which are described below, is met or if at least one of the first condition or the second condition and a third condition are met.

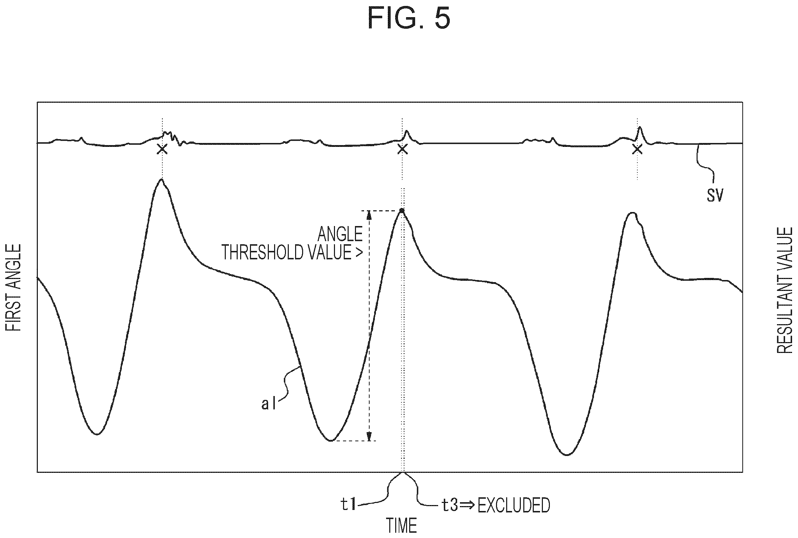

The first condition is detection of the third time t3 based on the first time t1 corresponding to a local maximum value at which a change to the local maximum value immediately after a local minimum value of the first angle al is lower than an angle threshold value, as illustrated in FIG. 5. The angle threshold value is, for example, 50°. The control unit 18 may exclude the third time t3 when the first condition is met from the start time of one step in normal gait.

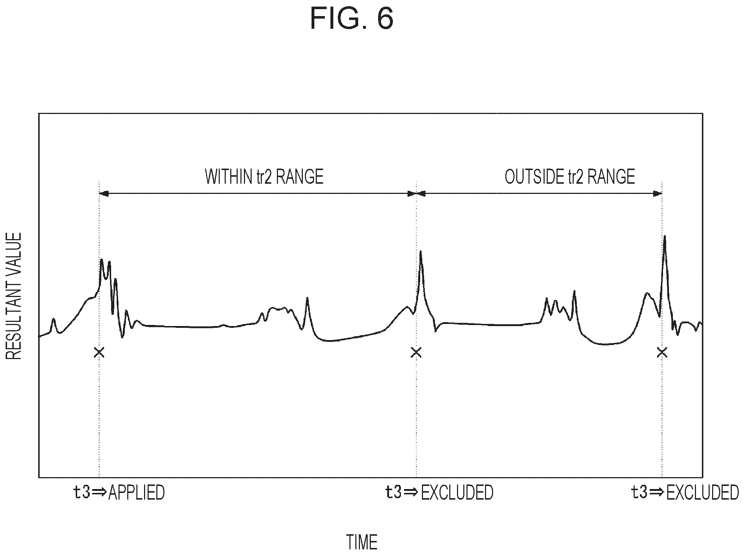

The second condition is that the time interval between the continuous third times t3 is outside a second time range tr2, as illustrated in FIG. 6. The second time range tr2 is a time range supposed as a gait cycle in normal gait and is, for example, 0.5 ms ≤ the second time range tr2 ≤ 2.0 sec. The gait cycle is a cycle during which one foot lands on the ground, is swung forward and up, and lands on the ground again. The control unit 18 may exclude the third time t3 from the start time of one step in normal gait if the second condition is met.

**In** order to define the third condition, the control unit 18 may extract the remaining multiple third times t3 excluded from the start time based on at least one of the first condition or the second condition from the multiple third times t3 that have been detected with time. In addition, the control unit 18 may extract the other third times t3 resulting from further exclusion of the two third times t3 that have been first and secondly detected, among the remaining multiple third times t3. Furthermore, the control unit 18 may calculate the time intervals between the continuous third times t3, among the other third times t3. Furthermore, the control unit 18 may calculate an average value of the multiple time intervals. Furthermore, the control unit 18 determines whether each time interval is outside a third time range with respect to the average value. The third time range may be defined based on a statistical value of the time intervals used for the calculation of the average value. The third time range is, for example, a range of a standard deviation σ with respect to an average value µ. The third condition is deviation of the time interval from the third time range. The control unit 18 may exclude the third times t3 concerning the time interval when the third condition is met from the start time of one step in normal gait.

The control unit 18 may calculate a feature value based on the angular velocities and the accelerations at the wearing portion in the global coordinate system during a time from the certified start time of one step to the start time of the next step in normal gait to analyze the gait state of the wearer based on the feature value. The control unit 18 may cause the output unit 20 to output the result of analysis.

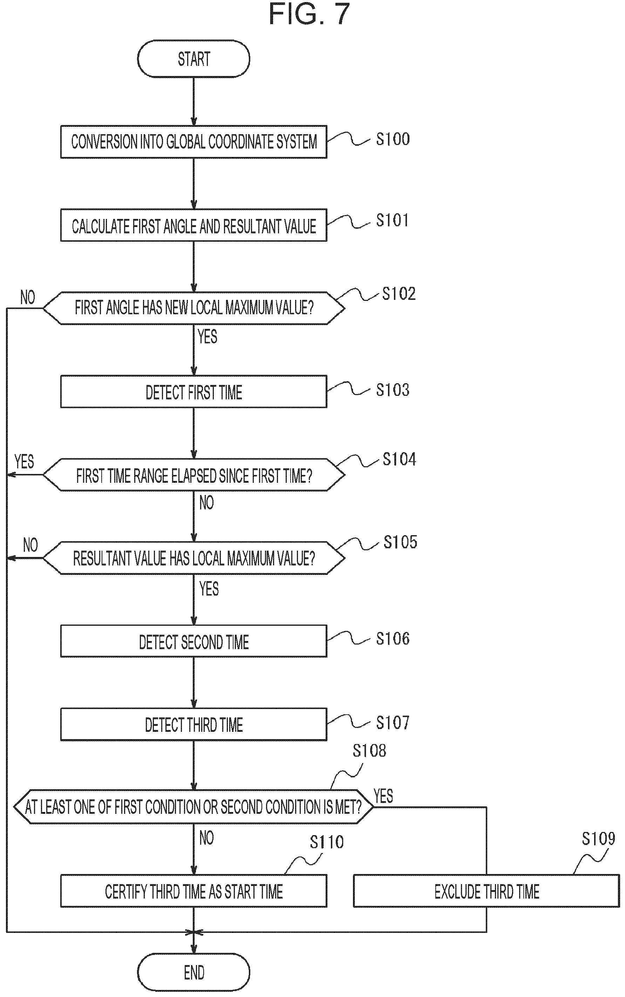

Next, a certification process performed by the control unit 18 in the information processing device 10 in the present embodiment will be described with reference to a flowchart in FIG. 7. The certification process is started each time the original accelerations and the original angular velocities are acquired from the sensor apparatus 12.

In Step S100, the control unit 18 converts the original accelerations and the original angular velocities in the local coordinate system into the accelerations and the angular velocities in the global coordinate system. After the conversion, the process goes to Step S101.

In Step S101, the control unit 18 calculates the first angle al and the resultant value sv using the accelerations and the angular velocities resulting from the conversion in Step S100. The control unit 18 stores the first angle al and the resultant value sv that are calculated in the storage unit 21 in association with time. After the calculation, the process goes to Step S102.

In Step S102, the control unit 18 determines whether the first angle al calculated at each time has a new local maximum value. The new local maximum value is the local maximum value of the first angle al, which is not used for the detection of the third time t3. If the new local maximum value does not exist, the certification process is terminated. If the new local maximum value exists, the process goes to Step S103.

In Step S103, the control unit 18 detects the time associated with the first angle al having the new local maximum value the existence of which is confirmed in Step S102 as the first time t1. After the detection, the process goes to Step S104.

In Step S104, the control unit 18 determines whether the first time range tr1 elapsed since the first time t1 detected in Step S103. If the first time range tr1 elapsed, the certification process is terminated. If the first time range tr1 did not elapse, the process goes to Step S105.

In Step S105, the control unit 18 determines whether the resultant value sv calculated at each time has a new local maximum value. The new local maximum value is the local maximum value of the resultant value sv, which is not used for the detection of the third time t3. If the new local maximum value does not exist, the certification process is terminated. If the new local maximum value exists, the process goes to Step S106.

In Step S106, the control unit 18 detects the time associated with the resultant value sv having the new local maximum value the existence of which is confirmed in Step S105 as the second time t2. After the detection, the process goes to Step S107.

In Step S107, the control unit 18 detects the third time t3 based on the second time t2 detected in Step S106 and the resultant value sv calculated at each time. After the detection, the process goes to Step S108.

In Step S108, the control unit 18 determines whether the third time t3 detected in Step S107 meets at least one of the first condition or the second condition. If the third time t3 detected in Step S107 meets at least one of the first condition or the second condition, the process goes to Step S109. If the third time t3 detected in Step S107 meets none of the first condition and the second condition, the process goes to Step S110.

In Step S109, the control unit 18 excludes the third time t3 detected in Step S107 from the start time. After the exclusion, the certification process is terminated.

In Step S110, the control unit 18 certifies the third time t3 detected in Step S107 as the start time. The control unit 18 stores the third time t3 certified as the start time in the storage unit 21. After the certification, the certification process is terminated.

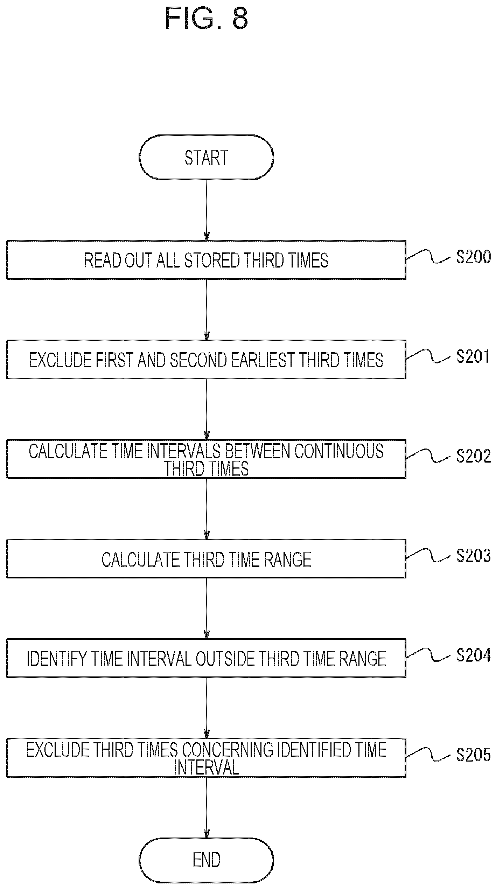

Next, an exclusion process performed by the control unit 18 in the information processing device 10 in the present embodiment will be described with reference to a flowchart in FIG. 8. The exclusion process may be started after the certification process is terminated or may be, for example, periodically started independently from the certification process.

In Step S200, the control unit 18 reads out all the third times t3 stored in the storage unit 21. After the readout, the process goes to Step S201.

In Step S201, the control unit 18 excludes the first and second earliest third times t3 from the third times t3 read out in Step S200. After the exclusion, the process goes to Step S202.

In Step S202, the control unit 18 extracts the remaining multiple third times t3 resulting from the exclusion in Step S202. In addition, the control unit 18 calculates the time intervals between the continuous third times t3, among the extracted third times t3. After the calculation, the process goes to Step S203.

In Step S203, the control unit 18 calculates the third time range based on the time intervals calculated in Step S202. After the calculation, the process goes to Step S204.

In Step S204, the control unit 18 identifies the time interval outside the third time range calculated in Step S203, among the time intervals calculated in Step S202. After the identification, the process goes to Step S205.

In Step S205, the control unit 18 excludes the third times t3 concerning the time interval identified as being outside the third time range in Step S204. The control unit 18 cancels the certification of the excluded third times t3 as the start time. After the cancellation, the exclusion process is terminated.

The information processing device 10 of the present embodiment, which has the configuration described above, certifies the start of one step in gait of the wearer of the inertial sensor using the first angle al based on the original angular velocities and the resultant value sv based on the original accelerations. With such a configuration, since the information processing device 10 acquires the original angular velocities and the original accelerations from the mobile sensor apparatus 12, the information processing device 10 is capable of detecting the start time of one step in gait with no limitation of the location. In addition, with such a configuration, the information processing device 10 can have improved detection accuracy of the start time of one step. Accordingly, the information processing device 10 can have improved detection accuracy of the operation in gait with no limitation of the location.

Furthermore, the information processing device 10 detects the second time t2 at which the resultant value sv is locally maximized within the first time range tr1 from the first time t1 at which the first angle al is locally maximized and certifies the third time t3 at which the resultant value sv is locally minimized toward the first time t1 from the second time t2 as the start of one step. In gait, the moment at which the foot is swung forward and up and lands on the ground immediately after the first angle al is locally maximized is the start point of time of one step. Accordingly, the inventor of the present application estimates that the foot does not land on the ground at the point of time at which the first angle al is locally maximized. In addition, the inventor of the present application also estimates that the acceleration of the ankle is gradually increased from the landing point of time to reach a peak. In consideration of the above estimation, since the information processing device 10 having the above configuration detects the time immediately before the landing, at which the first angle al is locally maximized, and estimates the time at which the acceleration is locally minimized immediately before the acceleration is locally maximized from the time, the information processing device 10 can have improved detection accuracy of the start time of one step.

Furthermore, the information processing device 10 excludes the third time t3 based on the first time t1 at which a change to the local maximum value immediately after the local minimum value of the first angle al is lower than the angle threshold value from the start of one step. When the change from the local minimum value to the local maximum value of the first angle al is small, the movement of the foot while the change from the local minimum value to the local maximum value of the first angle al is small is generally a movement, such as jumping, skipping, start of gait, deceleration, turning around, or stepping, which is different from the normal gait. Accordingly, since the information processing device 10 having the above configuration excludes the third time t3 that is detected in association with the operation state different from the normal gait, the information processing device 10 is capable of detecting the start time of one step in normal gait.

Furthermore, the information processing device 10 excludes the third times t3 from the start of one step if the time interval between the continuous third times t3 is outside the second time range tr2. The gait cycle in normal gait, that is, the continuous third times t3 are 1.29 seconds and the time interval that is extremely different from the gait cycle is the interval when the operation different from the normal gait is performed. For example, the gait cycle when a turning around operation is performed is generally shorter than the gait cycle of the normal gait. For example, the gait cycle when a stopping operation is performed is generally longer than the gait cycle of the normal gait. Accordingly, since the information processing device 10 having the above configuration excludes the third times t3 detected in association with the operation state different from the normal gait, the information processing device 10 is capable of detecting the start time of one step in normal gait.

Furthermore, the information processing device 10 extracts the remaining third times t3 excluded from the start of one step, among the multiple third times t3, extracts the other third times t3 resulting from further exclusion of the two third times t3 that have been first and secondly detected, among the remaining third times t3, calculates the average value of the time intervals between continuous third times t3, among the other third times t3, and further excludes the third times t3 concerning the time interval outside the third time range with respect to the average value from the start of one step. With such a configuration, since the information processing device 10 excludes the third times t3, which are abnormal values, from the state in which the stable normal gait is estimated to be performed, the information processing device 10 can have improved accuracy of the start time of one step in normal gait.

Furthermore, the information processing device 10 converts the original accelerations and the original angular velocities into the accelerations and the angular velocities in the global coordinate system including the vertical direction as the coordinate axis based on the change in posture with respect to the initial posture. Although the ideal wearing posture is supposed for the sensor apparatus 12, the practical wearing posture is generally shifted from the ideal wearing posture. Against such an event, since the first angle al comes close to the angle of the direction in which the tibia of the wearer extends with respect to the vertical direction in the information processing device 10 having the above configuration, reduction in the detection accuracy of the start time is capable of being reduced even if the practical wearing posture is shifted from the ideal wearing posture.

### EXAMPLES

Multiple ground reaction force sensors were laid on the floor on a plane in a matrix to provide an actual measurement area. When a subject was caused to walk in the actual measurement area, the point of time at which the sensor value output from the ground reaction force sensor rises from zero was considered as the landing point of time of a foot, which was assumed as the start time of one step.

The sensor apparatus including the 3-axis inertial sensor was mounted to an ankle of the subject. The third times were detected based on the coordinate conversion, the detection of the first time, and the detection of the second time, which are described in the above embodiment, using the original accelerations and the original angular velocities output from the sensor apparatus during the gait in the actual measurement area. The first time range tr1 was set to 100 ms.

The start times, which were detected by the ground reaction force sensor during the gait in the actual measurement area, and the third times and the time intervals, which were based on the original accelerations and the original angular velocities output from the sensor apparatus, were calculated. When the gait in the actual measurement area was performed 21 times, µ ± σ was -23.8 ms ± 22.4 ms where the average value and the standard deviation of the time intervals calculated in the respective gaits were denoted by µ and σ, respectively. Since the time before the impact caused by the landing of the heel reached the sensor apparatus mounted to the ankle was 20 ms to 30 ms, the differences between the third times detected by the above method and the actual landing times were small.

It was determined whether the third time that was detected using the original accelerations and the original angular velocities output from the sensor apparatus when turning around was performed in the actual measurement area is excluded based on the first condition to the third condition. The angle threshold value in the first condition was set to 50°. The second time range in the second condition was set to a value that is longer than or equal to 0.5 ms and that is shorter than or equal to 2.0 sec. The third time range in the third condition was set to µ ± σ. In the gait when the turning around was performed, the third time was excluded. Accordingly, at least turning around, which is abnormal gait, was excluded from the certification of the start time.

In one embodiment, (1) an information processing device includes
an acquisition unit configured to acquire an original acceleration and an original angular velocity in a local coordinate system based on a posture of a 3-axis inertial sensor from the inertial sensor, and
a control unit configured to certify start of one step in gait of a wearer of the inertial sensor using a first angle with respect to a vertical direction based on the original angular velocity and a resultant value based on the original acceleration.

(2) In the information processing device in (1) described above,
   the control unit detects a second point of time at which the resultant value is locally maximized within a first time range from a first point of time at which the first angle is locally maximized and certifies a third point of time at which the resultant value is locally minimized toward the first point of time from the second point of time as the start of one step.
(3) In the information processing device in (2) described above,
   the control unit excludes the third point of time based on the first point of time at which a change to a local maximum value immediately after a local minimum value of the first angle is lower than an angle threshold value from the start of one step.
(4) In the information processing device in (2) or (3) described above,
   the control unit excludes the third points of time from the start of one step if a time interval between continuous third points of time is outside a second time range.
(5) In the information processing device in (3) or (4) described above,
   the third point of time includes multiple third points of time, and
   the control unit extracts remaining third points of time resulting from exclusion from the start, among the multiple third points of time, extracts the other third points of time resulting from further exclusion of two third points of time that have been first and secondly detected, among the remaining third points of time, calculates an average value of time intervals between continuous third points of time, among the other third points of time, and further excludes the third points of time concerning the time interval outside a third time range with respect to the average value from the start of one step.
(6) In the information processing device in (1) to (5) described above,
   the control unit converts the original acceleration and the original angular velocity into an acceleration and an angular velocity, respectively, in a global coordinate system including the vertical direction as a coordinate axis based on change in posture with respect to an initial posture.
(7) In the information processing device in (1) to (6) described above,
   the acquisition unit acquires the original acceleration and the original angular velocity through wireless communication from the 3-axis inertial sensor provided in a sensor apparatus of a type mounted to a leg.

In one embodiment, (8) an information processing method includes
acquiring an original acceleration and an original angular velocity in a sensor coordinate system from a 3-axis inertial sensor, and
certifying start of one step in gait of a wearer of the inertial sensor using a first angle with respect to a vertical direction based on the original angular velocity and a resultant value based on the original acceleration.

Although the embodiment of the information processing device 10 is described above, a mode of a storage medium on which programs are recorded (for example, an optical disk, a magneto-optical disk, a CD-ROM (compact disk-read only memory), a CD-R (compact disk-recordable), a CD-RW (compact disk-rewritable), a magnetic tape, a hard disk, or a memory card) is applicable, in addition to a method or a program for embodying the apparatus, as the embodiment of the present disclosure.

The embodiment of the program is not limited to an application program, such object code compiled by a compiler or program code executed by an interpreter. The program may be embodied as a program module incorporated in an operating system or the like. In addition, the program may be configured or may not be configured so that all processing is executed only on a CPU (central processing unit) on a control board. Part of the program or the entire program may be executed by another processing unit installed in an extension board or an extension unit, which is added to the board, if needed.

The drawings describing the embodiments according to the present disclosure are schematically illustrated. The ratios of the sizes on the drawings and so on do not necessarily coincide with the actual ones.

Although the embodiments of the present disclosure are described above based on the drawings and the examples, it is to be noted that various variations or modifications are available by persons skilled in the art based on the present disclosure. Accordingly, it is to be noted that the variations and modifications are included in the scope of the present disclosure. For example, the functions and so on included in the respective components and so on are capable of being rearranged with no logical inconsistency. The multiple components and so on may be integrated into one or may divided.

For example, although the information processing device 10 is provided separately from the sensor apparatus 12, the information processing device 10 may be incorporated in the sensor apparatus 12.

All the components described in the present disclosure and/or all the disclosed methods or all the steps in the processes may be arbitrarily combined except the combinations in which the features are mutually exclusive. The respective features described in the present disclosure may be replaced with alternate features functioning for the same purposes, equivalent purposes, or similar purposes unless explicitly negated. Accordingly, the respective features that are disclosed are only examples of a series of comprehensive same or equivalent features unless explicitly negated.

In addition, the embodiments according to the present disclosure are not restricted by any specific component in the embodiments described above. The embodiments according to the present disclosure can be extended to all the new features described in the present disclosure or combinations of the features, or all the new methods that are described, the steps in the processes, or combinations of the methods and the steps in the processes.

In the present disclosure, "first", "second", and so on that are described are identifiers for identifying the corresponding components. The numbers of the components identified by the description of "first", "second", and so on in the present disclosure may be replaced with each other. For example, the identifier "first" of a first information processing device may be replaced with the identifier "second" of a second information processing device. The replacement of the identifiers is simultaneously performed. The components are discriminated after the replacement of the identifiers. The identifiers may be deleted. The components from which the identifiers are deleted are discriminated with signs. The description of the identifiers, such as "first" and "second", in the present disclosure is not used as the basis of interpretation of the order of the components and existence of smaller-number identifiers.

### REFERENCE SIGNS

- 10: information processing device
- 11: information processing system
- 12: sensor apparatus
- 13: communication unit
- 14: sensor unit
- 15: storage unit
- 16: control unit
- 17: communication unit
- 18: control unit
- 19: input unit
- 20: output unit
- 21: storage unit
- al: first angle
- sv: resultant value
- t1: first time
- t2: second time
- tr1: first time range
- tr2: second time range

## Claims

1. An information processing device comprising:
an acquisition unit configured to acquire an original acceleration and an original angular velocity in a local coordinate system based on a posture of a 3-axis inertial sensor from the inertial sensor; and
a control unit configured to certify start of one step in gait of a wearer of the inertial sensor using a first angle with respect to a vertical direction based on the original angular velocity and a resultant value based on the original acceleration.

2. The information processing device according to claim 1,
wherein the control unit detects a second point of time at which the resultant value is locally maximized within a first time range from a first point of time at which the first angle is locally maximized and certifies a third point of time at which the resultant value is locally minimized toward the first point of time from the second point of time as the start of one step.

3. The information processing device according to claim 2,
wherein the control unit excludes the third point of time based on the first point of time at which a change to a local maximum value immediately after a local minimum value of the first angle is lower than an angle threshold value from the start of one step.

4. The information processing device according to claim 2 or 3,
wherein the control unit excludes the third points of time from the start of one step if a time interval between continuous third points of time is outside a second time range.

5. The information processing device according to claim 3 or 4,
wherein the third point of time comprises a plurality of third points of time, and
wherein the control unit extracts remaining third points of time resulting from exclusion from the start of one step, among the plurality of third points of time, extracts the other third points of time resulting from further exclusion of two third points of time that have been first and secondly detected, among the remaining third points of time, calculates an average value of time intervals between continuous third points of time, among the other third points of time, and further excludes the third points of time concerning the time interval outside a third time range with respect to the average value from the start of one step.

6. The information processing device according to any of claims 1 to 5,
wherein the control unit converts the original acceleration and the original angular velocity into an acceleration and an angular velocity, respectively, in a global coordinate system including the vertical direction as a coordinate axis based on change in posture with respect to an initial posture.

7. The information processing device according to any of claims 1 to 6,
wherein the acquisition unit acquires the original acceleration and the original angular velocity through wireless communication from the 3-axis inertial sensor provided in a sensor apparatus of a type mounted to a leg.

8. An information processing method comprising:
acquiring an original acceleration and an original angular velocity in a sensor coordinate system from a 3-axis inertial sensor; and
detecting start of one step in gait of a wearer of the inertial sensor using a first angle with respect to a vertical direction based on the original angular velocity and a resultant value based on the original acceleration.
